# FASCICULE DE BREVET EUROPEEN

(11) **EP 0 532 405 B1**
(45) Date de publication et mention de la délivrance du brevet: **03.01.1996**
(21) Numéro de dépôt: 92402452.4
(22) Date de dépôt: 08.09.1992
(51) Int. Cl.: A47G 25/90, B25J 21/02, A61B 19/04, G21F 7/053

(54) **Dispositif pour retirer des gants contaminés**
Vorrichtung zum Ausziehen von verunreinigten Handschuhen
Device for taking off contaminated gloves

(30) Priorité: 10.09.1991 FR 9111143
(43) Date de publication de la demande: 17.03.1993
(73) Titulaire: COMMISSARIAT A L'ENERGIE ATOMIQUE, F-75015 Paris (FR)
(72) Inventeur: Counit, Georges, F-26700 La Garde Adhemar (FR); Charvolin, Michel, F-21240 Talant (FR)
(74) Mandataire: Dubois-Chabert, Guy

(56) Documents cités:
- EP-A- 0 219 138
- WO-A-90/06730
- FR-A- 2 614 517
- US-A- 4 915 272

## Description

La présente invention concerne un dispositif pour retirer des vêtements de protection, tels que des gants, qui ont été contaminés par contact avec des produits ou des objets toxiques.

L'invention trouve des applications dans de nombreux domaines industriels, tels que le nucléaire, la chimie et l'agro-alimentaire. Elle peut également s'appliquer au domaine de la santé publique, dans lequel le personnel médical peut se trouver au contact de substances minérales ou organiques agressives et/ou toxiques.

Dans tous ces domaines, il est souvent nécessaire de protéger les membres des opérateurs et, plus particulièrement, leurs mains avec des protections, telles que des gants. Différentes sortes de gants peuvent être utilisées selon les caractéristiques d'agressivité des produits et objets à manipuler : ces gants peuvent tout aussi bien être des gants noirs de vinyle épais que des gants de latex blanc très mince ou encore des gants en caoutchouc d'épaisseur moyenne.

L'utilisation de ces gants pose cependant un problème, lorsqu'il s'agit de les retirer sans risque de toucher la peau avec la surface contaminée du gant.

Dans la plupart des cas, l'opérateur enlève un premier gant et se sert alors de sa première main libérée pour retirer le second gant. Selon les consignes, l'opérateur doit retourner progressivement le gant en le repliant sur lui-même de manière à enfermer la contamination. Toutefois, cette opération est longue et délicate. Bien souvent, l'opérateur, pressé par le temps, ne respecte pas, ou du moins pas totalement, les consignes prescrites pour l'enlèvement des protections et se contamine les mains.

En plus des risques inhérents à la santé du travailleur, la contamination peut s'étendre par contact à l'environnement (par exemple : rampe d'escalier, objets divers manipulés), ce qui nécessite des opérations de décontamination longues et coûteuses.

Un dispositif pour enlever des gants a déjà été décrit dans un document français, déposé au nom du demandeur et publié sous le numéro FR-2 601 492.

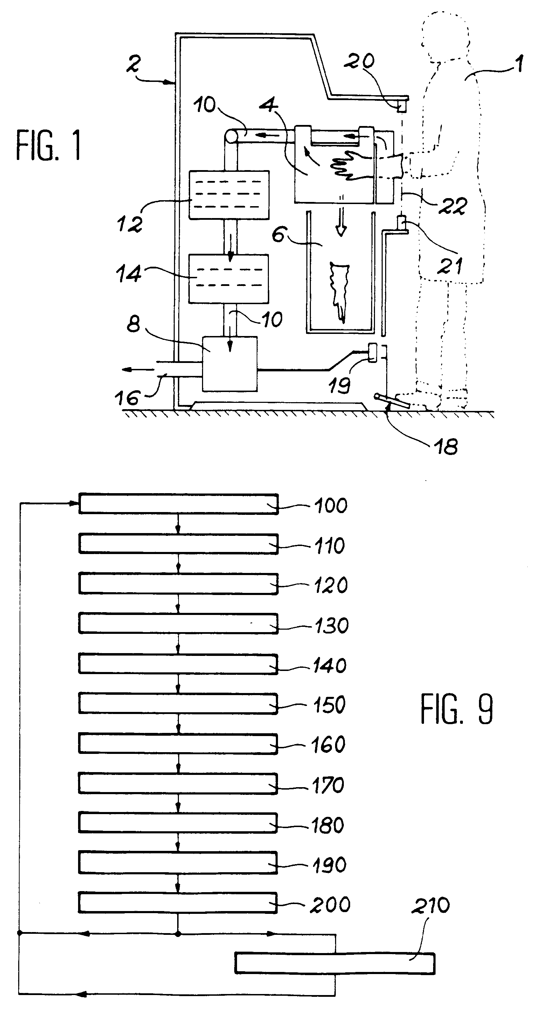

Sur la figure 1, on a représenté ce dispositif selon l'art antérieur. Il est constitué, d'une manière générale, d'un carter 2 comprenant une boîte 4 d'enlèvement des protections (enlèvement des gants), d'un réceptacle 6 de récupération des protections retirées et des moyens 8 pour créer une dépression à l'intérieur de ladite boîte 4 d'enlèvement. Ces moyens 8 de dépression sont reliés à la boîte 4 d'enlèvement par l'intermédiaire d'une canalisation 10 traversant elle-même des filtres 12 et 14 ayant pour rôle de piéger la contamination qui s'est déposée sur la surface extérieure du gant au cours des manipulations. Les moyens 8 de dépression, pour créer une telle dépression, aspirent l'air présent dans la boîte 4 d'enlèvement et rejettent cet air, qui a été décontaminé par les filtres 12 et 14, par une canalisation 16 donnant sur l'extérieur du dispositif.

Ces moyens 8 de dépression sont également reliés à un dispositif 18, 19 électrique permettant de commander lesdits moyens 8 de dépression. Selon le dispositif de l'art antérieur, ce dispositif électrique comprend une pédale 18 actionnée par l'opérateur pour commander la mise en marche de la machine d'enlèvement des protections. Cette pédale 18 est reliée à un interrupteur 19 qui, lorsqu'il est fermé, permet d'alimenter en courant électrique les moyens 8 de dépression. A l'inverse, lorsque l'opérateur relâche la pression sur la pédale 18, l'interrupteur 19 s'ouvre, le courant est coupé et l'aspiration par les moyens 8 de dépression est arrêtée.

Cette boîte 4 d'enlèvement des protections comporte de façon schématique une paroi perforée qui divise le volume de ladite boîte en deux chambres, à savoir une chambre de dépression et une chambre de réception dont la forme se rapproche le plus possible de celle d'une main. Lors de la mise en marche des moyens 8 de dépression, une dépression est créée dans la chambre de dépression de la boîte 4 d'enlèvement, cette dépression ayant pour effet de décoller le gant de la main de l'opérateur.

La boîte 4 d'enlèvement a pour caractéristique de s'ouvrir selon une articulation horizontale, ce qui permet au gant retiré de la main de l'opérateur de tomber dans le réceptacle 6 placé physiquement sous ladite boîte 4 d'enlèvement des gants. Le principe de la chute libre est donc utilisé pour récupérer les gants retirés dans le réceptacle 6.

Une source lumineuse 20 émet un faisceau lumineux 22 vers un réflecteur 21 qui renvoit ce faisceau sur l'élément récepteur de la cellule. Lorsque le faisceau 21 est interrompu (présence des mains de l'opérateur), la mise en route des moyens d'aspiration 8 s'effectue selon une temporisation prédéfinie, pour permettre respectivement l'introduction et l'enlévement des mains de l'opérateur.

Ce dispositif selon l'art antérieur a pour inconvénient, notamment, de nécessiter une boîte d'enlèvement des protections ayant une forme très similaire à la forme d'une main. De plus, elle nécessite l'ouverture de cette boîte d'enlèvement pour faire tomber le gant dans le réceptacle. Par ailleurs, il s'avère, dans certains cas, que le gant se retourne sur la main lorsque l'opérateur appuie sur la pédale de fonctionnement au moment où le gant est introduit dans la boîte d'enlèvement des gants.

Un autre dispositif pour retirer des gants est décrit dans le document US-A-4 915 272. Selon une réalisation décrite dans ce document, le dispositif pour retirer les gants comporte un cylindre dans lequel l'utilisateur introduit sa main gantée. Des segments disposés à l'entrée du cylindre sont rabattus vers les parois du cylindre lors de l'introduction de la main gantée dans le cylindre. Lorsque la main est totalement introduite, les segments se rabattent sur le poignet de l'utilisateur. Aussi, lorsque ledit utilisateur retire sa main du cylindre, les segments attrapent le bourrelet du gant et le maintiennent dans le cylindre pendant que l'utilisateur retire sa main. Selon une autre variante de ce dispositif pour retirer les gants, ledit dispositif comporte un cylindre soumis à une pression négative (par rapport à la pression régnant à l'extérieur du dispositif) et un diaphragme en caoutchouc situé à l'entrée dudit cylindre. Lorsque l'utilisateur a introduit sa main gantée dans le cylindre, la pression régnant dedans a pour effet de décoller le gant de la main.

Ce dispositif comporte donc un diaphragme ne pouvant prendre qu'une position ouverte ou fermée, ce qui présente l'inconvénient d'une mauvaise adaptation autour du poignet et donc d'une mauvaise étanchéité du gant.

Le document EP-A-219 138 décrit un container pour cosmétiques ayant un diaphragme pouvant prendre plusieurs positions.

La présente invention a justement pour objet de remédier à ces inconvénients en proposant notamment des moyens pour retenir le gant sur le bras afin qu'il ne se retourne pas à la mise en fonctionnement du dispositif, ces moyens assurant de plus une étanchéité quasi-parfaite entre la boîte d'enlèvement et l'extérieur. De plus, l'invention propose un système d'aspiration des gants dans le réceptacle, ce système utilisant les moyens connus de dépression.

De façon plus précise, l'invention est décrite dans le revendication principen.

Ce dispositif se caractérise par le fait qu'il comprend des moyens d'étanchéité de la boîte d'enlèvement par rapport à l'extérieur, lesdits moyens agissant par pression sur le membre revêtu du vêtement de protection à retirer.

Avantageusement, les moyens d'étanchéité comportent au moins un diaphragme de forme adaptable au contour du membre revêtu du vêtement de protection.

Ce diaphragme peut prendre trois positions :
- une position de fermeture, dans laquelle il enserre le membre revêtu du vêtement de protection afin d'obtenir l'étanchéité entre la boîte d'enlèvement et l'extérieur ;
- une position d'ouverture, dans laquelle il permet le passage du membre vers ou hors de la boîte d'enlèvement ; et
- une position intermédiaire de transition, dans laquelle il permet un passage contrôlé de l'air entre l'extérieur du membre et l'intérieur du vêtement de protection.

En outre, le dispositif selon l'invention comprend des moyens pour aspirer les vêtements retirés vers le réceptacle. Ces moyens comportent un obturateur amovible, situé à la jointure de la chambre de réception et de la canalisation, et assurant, dans une première position, une fermeture entre ladite chambre de réception et ladite canalisation et, dans une seconde position, une ouverture entre lesdites chambre et canalisation.

Pour un meilleur confinement dans la boîte d'enlèvement, les moyens pour aspirer comportent en outre un opercule, commandé par un moteur, permettant de fermer hermétiquement la boîte d'enlèvement lors de l'aspiration des vêtements vers le réceptacle.

De façon avantageuse, les moyens pour aspirer les vêtements de protection comportent en outre une vanne assurant un passage de l'air entre l'extérieur et la boîte d'enlèvement, pour la mise à pression normale dans ladite boîte.

Selon un mode de réalisation de l'invention, la canalisation comporte, à son embouchure dans le réceptacle, un compteur de vêtements relié électriquement aux moyens de commande des moyens de dépression pour prévenir lorsque le réceptacle est plein.

D'autres avantages et caractéristiques de l'invention apparaitront au cours de la description qui va suivre, donnée à titre illustratif, mais nullement limitatif, en référence aux dessins dans lesquels :
- la figure 1, déjà décrite, représente le dispositif d'enlèvement des gants selon l'art antérieur ;
- la figure 2 est une représentation schématique du dispositif pour retirer des protections correspondant à l'invention ;
- la figure 3 est une représentation vue de face, carter de façade enlevé, du dispositif représenté sur la figure 2 ;
- la figure 4 est une représentation schématique de la partie supérieure du dispositif, c'est-à-dire de l'ensemble constitué de la boîte d'enlèvement et des moyens d'aspiration directement liés à ladite boîte ;
- la figure 5 représente schématiquement la boîte d'enlèvement dans laquelle l'utilisateur introduit sa main gantée ;
- la figure 6 représente la même boîte d'enlèvement que la figure 5, boîte dans laquelle le gant se décolle de la main de l'utilisateur ;
- la figure 7 représente cette même boîte d'enlèvement dans laquelle le gant s'est décollé de la main et où le diaphragme s'ouvre afin que l'utilisateur puisse retirer sa main de la machine ;
- la figure 8 représente la boîte d'enlèvement lorsque le gant est aspiré vers le réceptacle ;
- la figure 9 est une représentation fonctionnelle du procédé utilisé pour enlever les gants des mains d'un utilisateur.

Durant toute la description qui va suivre, on prendra, comme exemple de vêtements de protection, des gants, étant entendu que ce dispositif peut être réalisé de façon particulière pour d'autres sortes de vêtements de protection.

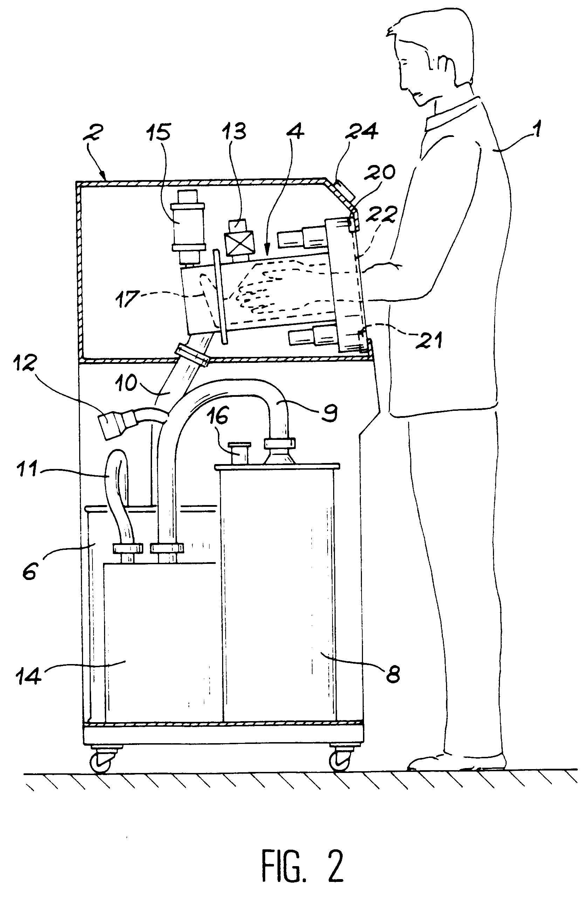

Sur la figure 2, on a représenté le dispositif selon l'invention. Les références données lors de la description de la figure 1 seront conservées pour les éléments identiques représentés sur la figure 2 et sur les figures suivantes. Ce dispositif selon l'invention est représenté sur cette figure de profil.

Un opérateur 1 introduit l'une de ses mains gantées dans la boite 4 d'enlèvement des gants. Cette boîte 4 est reliée par l'intermédiaire de la canalisation 10, du réceptacle 6 et de la canalisation 11, à un filtre 14 de décontamination lui-même relié par la canalisation 9 aux moyens 8 de dépression.

Ces moyens 8 de dépression peuvent comporter, par exemple, une turbine de laquelle débouche la canalisation 16 d'évacuation de l'air en provenance de la boîte 4 d'enlèvement des gants.

Selon le mode de réalisation décrit de l'invention, cet air évacué est décontaminé car passé par le filtre 14 de décontamination. Ce filtrage d'air est particulièrement intéressant car il permet de piéger la contamination qui s'est déposée sur la surface extérieure du gant au cours des manipulations effectuées par l'opérateur. De cette manière, l'air aspiré peut être rejeté sans risque hors de la machine par la canalisation 16.

Selon un autre mode de réalisation, visant à simplifier le dispositif selon l'invention, il est possible de ne pas introduire ce filtre 14, l'air étant alors directement aspiré par les moyens 8 de dépression et rejeté par la canalisation 16.

La canalisation 10 d'aspiration est également reliée, par une de ses extrémités, à la boîte 4 d'enlèvement des gants, et par son autre extrémité, au réceptacle de gants 6. Le rôle de cette canalisation 10 est essentiellement de permettre l'évacuation des gants retirés et de les conduire jusqu'au réceptacle 6. Son fonctionnement est lié au fonctionnement des moyens 13, 15 et 17 d'aspiration des gants, qui sera décrit ultérieurement.

Sur cette figure 2, un tableau de signalisation 24 permet l'affichage d'informations destinées à l'opérateur. Ces signaux, en effet, informent l'opérateur des possibilités qu'il a de mettre ou de retirer sa main (ou ses mains) gantée(s) ou dégantée(s) de la boîte 4 d'enlèvement des gants.

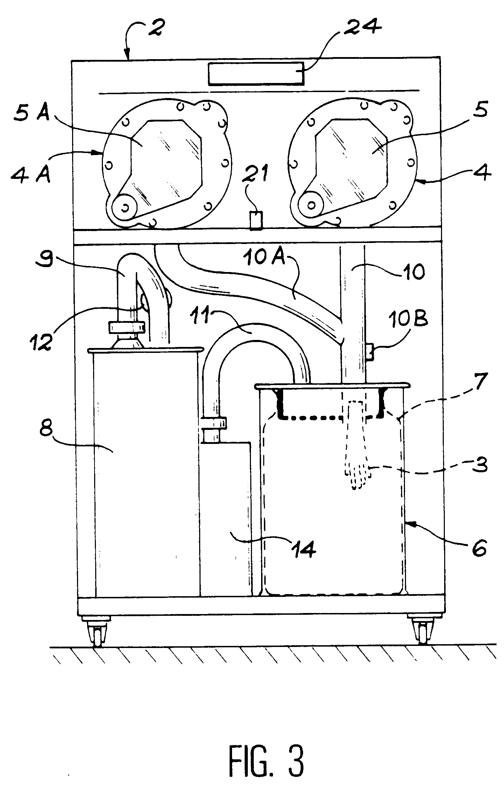

La figure 3 représente le dispositif selon l'invention, vue de face. On retrouve sur cette figure la plupart des éléments constitutifs dudit dispositif et déjà représentés sur la figure 2.

En effet, on voit sur cette figure, la boîte 4 d'enlèvement vue de face, c'est-à-dire qu'on voit plus précisément l'opercule 5 permettant la fermeture de la boîte 4 d'enlèvement et donc, le confinement d'une dépression d'air à l'intérieur de ladite boîte.

Selon le mode de réalisation exprimé sur cette figure 3, deux boîtes 4 et 4A d'enlèvement sont représentées et donc, deux opercules 5 et 5A. En effet, l'existence de deux boîtes 4 et 4A d'enlèvement de gants permet à l'opérateur de retirer ces deux gants de façon quasi-simultanée. Elle permet donc de gagner un temps considérable lorsque plusieurs opérateurs désirent retirer leurs gants dans un même intervalle de temps.

Il est cependant bien évident que le dispositif peut ne comporter qu'une seule boîte 4 d'enlèvement pour certains modes de réalisation.

De même, un tel dispositif pourrait comporter plus de deux boîtes d'enlèvement. Il est en effet possible de réaliser, par exemple, un dispositif comportant deux boîtes d'enlèvement de gants et deux boîtes d'enlèvement de surbottes.

Sur cette figure 3, sont également représentés les moyens 8 de dépression, ainsi que les canalisations 10 et 10A d'aspiration des gants vers le réceptacle 6. La canalisation 10A reliée à la boîte 4A d'enlèvement des gants rejoint la canalisation 10 en un plan de joint sous lequel est positionné un détecteur 10B de passage des gants. Ainsi, une seule canalisation (la canalisation 10 sur la figure), pourvue du détecteur 10B, débouche directement dans le réceptacle 6, ce qui permet le comptage des gants contenus dans ledit réceptacle 6.

En effet, selon un mode de réalisation, le détecteur 10B peut permettre, non seulement de totaliser le nombre de gants détectés et donc le nombre de gants contenus dans le réceptacle 6, mais aussi, de vérifier le passage des deux gants et, quand les deux gants ont été détectés, donner le signal de disponibilité de la machine.

De façon à protéger ledit réceptacle 6 de la contamination due au contact des gants, ce réceptacle 6 peut contenir un sac souple 7, par exemple un sac en vinyle, qui peut être entièrement retiré lorsqu'il est rempli de gants et remplacé par un autre sac 7 vide. Cette opération de changement de sac 7 est déclenchée par le comptage de gants et une signalisation optique ou sonore. Sur la figure 3, on montre justement un gant 3 en train de tomber dans le sac 7 de protection du réceptacle 6.

On voit également sur cette figure, le tableau 24 de signalisation, ainsi qu'un détecteur 21 à infrarouge. Ce détecteur 21 est situé entre les deux opercules 5 et 5A. Ce détecteur 21 assure la mise en marche/arrêt automatique du dispositif et, plus précisément, de l'ouverture des opercules 5 et 5A qui agit sur une préventilation.

En effet, un moto-ventilateur 12, branché sur la canalisation 9 assure une préventilation, c'est-à-dire un flux d'air allant de l'extérieur vers l'intérieur de la boîte 4 d'enlèvement des gants afin d'éviter toute diffusion d'une pollution interne à la machine vers l'extérieur.

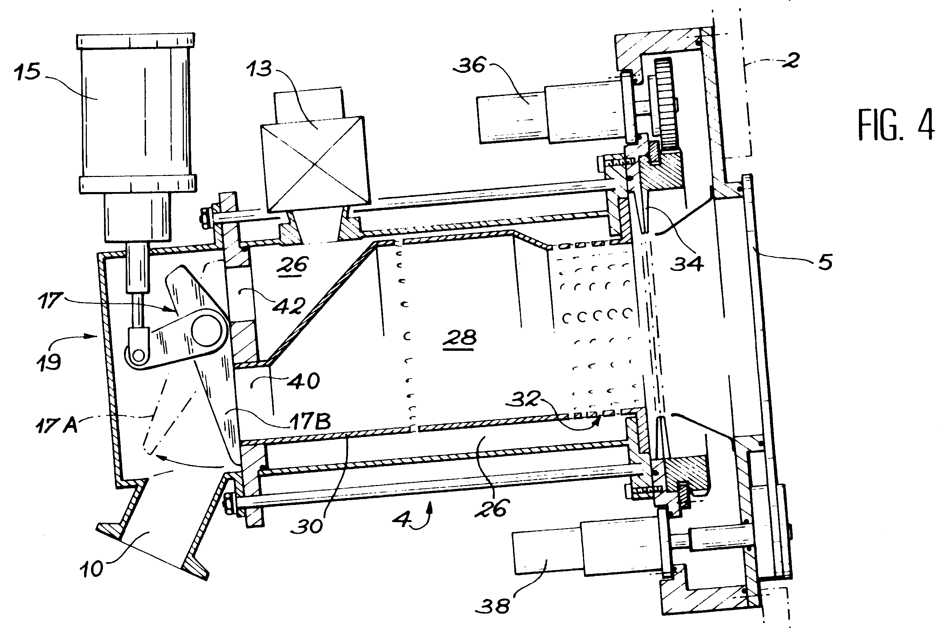

Sur la figure 4, on a représenté une vue en coupe de la partie supérieure du dispositif. Cette partie comporte notamment la boîte 4 d'enlèvement des gants, les moyens 13, 15 et 17 d'aspiration desdits gants et les moyens 34 d'étanchéité de la boîte 4.

La boîte 4 d'enlèvement des gants comprend une paroi 30 délimitant deux chambres : une chambre de réception 28 de la main gantée de l'opérateur et une chambre 26 de dépression. La chambre 26 est directement reliée à la canalisation 10 par laquelle l'air contenu dans la chambre 26 est aspiré afin que soit créé dans ladite chambre 26 une dépression. La paroi 30 est perforée de façon irrégulière, c'est-à-dire qu'elle contient une quantité de trous plus importante dans certaines zones que dans d'autres. En effet, la zone de la paroi correspondant au poignet de l'opérateur, lorsque celui-ci aura introduit sa main dans le dispositif, est plus perforée de façon à ce qu'un maximum de dépression pénètre à cet endroit dans la chambre 28 de réception. En effet, la dépression créée dans la chambre de dépression 26 pénètre dans la chambre 28 de réception par les perforations 32 fabriquées dans la paroi 30.

Afin de permettre un meilleur confinement de la dépression à l'intérieur de la boîte d'enlèvement 4, des moyens d'étanchéité 34 sont installés à l'entrée de ladite boîte 4. Ces moyens d'étanchéité comportent un diaphragme 34 qui se ferme sur le poignet de l'opérateur afin de permettre une étanchéité quasi parfaite de ladite boîte 4 d'enlèvement des gants.

Selon un autre mode de réalisation de l'invention, ces moyens d'étanchéité peuvent comporter un second diaphragme, monté en parallèle sur le diagramme 34, assurant l'étanchéité du dispositif lorsque le gant revêtu par l'opérateur est de taille supérieure à la taille standard, c'est-à-dire lorsque le gant monte plus haut que le poignet.

Ce diaphragme 34 peut prendre trois positions :
- une position d'ouverture, telle que montrée sur la figure 4, dans laquelle il est totalement ouvert et permet le libre passage de la main de l'opérateur ;
- une position de fermeture dans laquelle il permet de fermer hermétiquement l'entrée de la boîte 4, en se positionnant autour du poignet ganté de l'opérateur ;
- une position intermédiaire de transition -diaphragme en mouvement- dans laquelle le diaphragme est légèrement ouvert de façon à permettre aux gants de se décoller du poignet de l'opérateur, mais dans laquelle, également, il empêche l'opérateur de ressortir sa main du dispositif.

Comme on le voit sur la figure 4, ce diaphragme 34 a une section en forme de "V". Ce diaphragme est actionné à partir d'un moteur 36. Le rôle de ce diaphragme 34 sera décrit de façon plus détaillée dans la suite de la description.

Une boîte 19 contenant un obturateur 17 est fixée à l'arrière de la boîte 4 d'enlèvement des gants. Selon la position de cet obturateur, son rôle est de permettre la création d'une dépression dans la zone de dépression 26, ou bien de permettre l'aspiration du gant hors de la boîte 4 d'enlèvement du gant, lorsque la main de l'utilisateur est retirée dudit gant.

En effet, lorsque l'obturateur est dans une position 17B, il assure la fermeture 40 de la chambre 28 de réception et, en même temps, l'ouverture 42 de la chambre 26 de dépression. Dans cette position 17B, l'obturateur permet de créer la dépression dans la chambre 26 de dépression. Au contraire, dans sa position 17A, l'obturateur permet la fermeture du passage 42 de la chambre 26 de dépression et, en même temps, l'ouverture du passage 40 de la chambre 28 de réception. Dans cette position 17A, l'obturateur permet d'aspirer le gant, alors libéré de la main de l'opérateur, et se trouvant dans la chambre 28 de réception. Le mouvement de l'obturateur 17 est commandé à partir d'un dispositif 15 pouvant être, par exemple, un électro-aimant.

Afin de faciliter l'aspiration du gant dans la canalisation 10 (lorsque le gant a été libéré de la main de l'opérateur) une électro-vanne 13 est ouverte de façon à faire pénétrer, à l'intérieur de la boîte 4 d'enlèvement des gants, de l'air à la pression atmosphérique provenant de l'extérieur du dispositif. Cette introduction d'air à la pression atmosphérique permet de réaliser à l'intérieur de la boîte 4 d'enlèvement, une dépression permettant l'aspiration du gant dans la canalisation 10 reliée au réceptacle 6. L'ouverture de l'électro-vanne 13 débouche dans la chambre 26 de dépression. L'air ainsi introduit dans cette chambre 26 pénètre, par l'intermédiaire des perforations 32 de la paroi 30, à l'intérieur de la chambre de réception 28 poussant ainsi le gant libéré de la main de l'opérateur vers la canalisation 10.

Afin de faciliter le confinement de l'air dans la boîte 4 d'enlèvement des gants, un opercule 5, actionné par un moteur 38, vient fermer ladite boîte d'enlèvement 4 lorsque l'opérateur a retiré sa main.

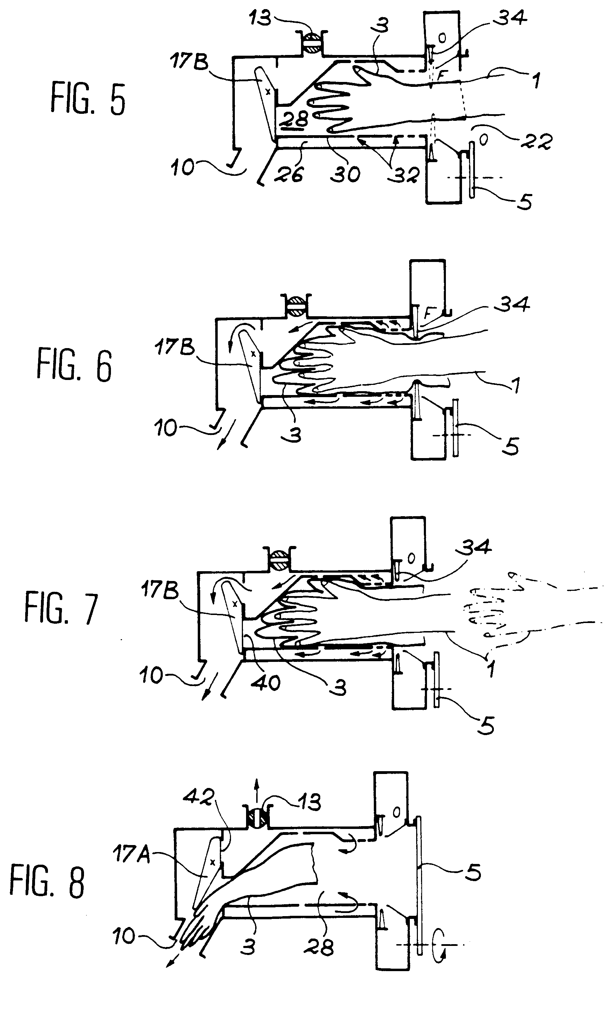

Sur les figures 5, 6, 7 et 8, on a représenté différentes étapes de l'enlèvement d'un gant.

Sur la figure 5, on voit l'opérateur 1 qui vient d'introduire sa main revêtue d'un gant 3 à l'intérieur de la boîte d'enlèvement 4. La pré-ventilation, qui se trouve à l'intérieur de ladite boîte, est alors arrêtée. De ce fait, il n'y a plus aucune dépression durant un temps prédéterminé, ce qui évite que le gant ne se retourne sur la main. Lorsque l'opérateur introduit sa main dans la boîte, le faisceau 22 est interrompu, ce qui a pour effet de provoquer le changement de position du diaphragme 34 qui passe de la position O d'ouverture à la position F de fermeture. Le diaphragme 34 vient alors se placer doucement autour du poignet. La fermeture de ce diaphragme 34 a pour effet d'assurer à l'intérieur de la boîte d'enlèvement 4 un confinement de la dépression d'air. L'électro-vanne 13 est fermée et l'obturateur 17 est en position 17B. Une valeur et un délai de fermeture prédéterminés du diaphragme 34 provoque la mise en service de la puissance maximum d'aspiration, c'est-à-dire de la puissance maximum des moyens de dépression. La dépression est alors rapidement obtenue dans la chambre de dépression 26 avec une étanchéité assurée par la fermeture du diaphragme 34. La dépression créée dans la chambre 26 se propage à travers les perforations 32 vers la chambre 28 de réception. Une dépression plus importante apparaît dans la zone du poignet de l'opérateur par l'intermédiaire de la paroi 30, plus perforée au niveau du poignet.

Comme montré sur la figure 6, le diaphragme s'ouvre alors dans sa position intermédiaire I durant la période de transition. La partie du gant qui est en contact avec le diaphragme 34 s'agrandit et va se plaquer contre la périphérie ovale de l'entrée de la boîte d'enlèvement 4. Cette dilatation du poignet du gant a pour effet d'isoler l'intérieur de la boîte d'enlèvement 4 par rapport à la partie extérieure de la machine. Sous l'effet de la différence de pression qui existe entre les volumes d'aspiration dans la chambre 28 de réception et l'intérieur du gant au niveau du poignet, la pression atmosphérique pénètre dans l'intérieur du gant et va plaquer celui-ci sur la paroi perforée 30. De façon plus précise, le gant 3 s'écarte progressivement de la paume de la main, puis du dos, et ensuite de chaque doigt.

Sur la figure 7, le diaphragme 34 s'est totalement ouvert et se trouve donc dans sa position d'ouverture O. L'opérateur peut ainsi retirer sa main de la boîte d'enlèvement 4. Ce geste est représenté sur la figure par la main en traits mixtes. Le retrait de la main rétablit le faisceau 22 qui provoque alors l'aspiration du gant vers le réceptacle.

Sur la figure 8, on a représenté cette aspiration du gant vers le réceptacle. En effet, lorsque l'opérateur a retiré sa main, que le faisceau 22 a été rétabli, le moteur 38 permet de refermer l'opercule 5 afin d'assurer un meilleur confinement à l'intérieur du dispositif. L'obturateur 17 passe alors dans sa position 17A afin de bloquer le passage 42 de la chambre de dépression 26 et d'ouvrir le passage 40 de la chambre de réception 28. L'électrovanne 13 est alors ouverte, permettant le passage de l'air sous pression atmosphérique vers la chambre de dépression 26. L'air engouffré dans cette chambre de dépression 26 va pénétrer à l'intérieur de la chambre de réception 28, grâce aux perforations de la paroi 30. Sous l'effet de cette pression, le gant va s'introduire dans la canalisation 10 et se diriger vers le réceptacle, non représenté sur la figure.

Selon un mode de réalisation de l'invention, et dans le but de faciliter l'aspiration du gant vers le réceptacle, la boîte d'enlèvement 4 peut être positionnée selon une légère inclinaison vers le bas en partant de l'entrée de la boîte d'enlèvement 4.

La commande de fermeture de l'opercule 5 est temporisée pour permettre l'aspiration du gant. Lorsque le gant est aspiré. L'opercule 5 s'ouvre à nouveau pour permettre l'introduction d'un nouveau bras dans le dispositif.

Sur la figure 9, on a représenté le diagramme fonctionnel du procédé utilisé pour enlever les gants d'un bras d'un opérateur.

Le bloc 100 représente une séquence d'initialisation, c'est-à-dire notamment de détection de la présence d'un bras vêtu d'un gant. Le bloc 110 représente la séquence d'ouverture des opercules et de la mise en marche des moyens de préventilation pour éviter toute contamination de l'intérieur vers l'extérieur de la machine. Le bloc 120 représente la séquence de temporisation permettant la mise en place des mains gantées à l'intérieur du dispositif. En 130, les préventilations sont arrêtées ; en 140, les diaphragmes sont fermés ; en 150, les moyens de dépression sont mis en marche pour créer la dépression à l'intérieur de la boîte d'enlèvement ; toute la séquence d'ouverture en position intermédiaire de diaphgrame et de décollement du gant s'effectue pendant cette séquence 150. Durant la séquence 160, les diaphragmes s'ouvrent et la signalisation "retirer les mains" est affichée sur le tableau de signalisation du dispositif afin que l'opérateur tienne compte de cet ordre et retire ses mains du dispositif. Le bloc 170 représente la séquence d'évacuation du gant droit avec la mise en marche des moyens d'aspiration des gants ; le passage du premier gant est détecté. Le bloc 180 représente la séquence d'évacuation du gant gauche avec la détection de passage du second gant. En 190, le dispositif est remis en état, c'est-à-dire que les moyens pour aspirer les gants vers le réceptacle sont arrêtés. Les opercules sont alors refermés. Dans le bloc 200, la préventilation est mise en marche et le nombre de gants présents dans le réceptacle est compté. Si ce nombre de gants présents dans le réceptacle n'atteint pas un nombre maximum préfixé, alors le procédé peut être réitérer et un second opérateur peut venir introduire ses mains dans le dispositif et le procédé reprend à l'étape 100. Au contraire, si le nombre de gants atteint le nombre maximum préfixé, alors une signalisation apparaît, demandant le changement du réceptacle, ou simplement le changement du sac en vinyl placé à l'intérieur du réceptacle. Lorsque le réceptacle a été vidé, le procédé peut être recommencé à partir de la séquence 100.

Selon l'invention, la forme de la chambre 28 de réception a une forme représentant grossièrement un contour de main. De ce fait, et afin de simplifier la fabrication du dispositif, il est tout à fait possible de considérer le contour des mains comme ambidextre, c'est-à-dire que le contour de la main droite est identique au contour de la main gauche.

Selon un mode de réalisation amélioré de l'invention, un système pour lacérer les gants est introduit à l'entrée du réceptacle. En effet, les gants retirés et aspirés de façon pneumatique, tel que décrit précédemment, contiennent des poches d'air. Le fait de lacérer ces gants, les viderait desdites poches d'air, ce qui permettrait le stockage d'un nombre plus important de gants pour un même volume de réceptacle.

## Revendications

1. Dispositif pour retirer un vêtement de protection enfilé sur un membre du corps d'un utilisateur, ce dispositif comprenant :
- au moins une boîte (4) d'enlèvement dudit vêtement comportant une chambre (28) de réception dans laquelle est introduit le membre revêtu du vêtement, une chambre (26) de dépression et une paroi (30) perforée située entre lesdites chambres ;
- des moyens (8) de dépression pour créer une différence de pression dans la chambre de réception par rapport à la pression normale extérieure à la boîte d'enlèvement ;
- un réceptacle (6), relié par une canalisation (10) à la chambre de réception, dans lequel sont stockés les vêtements de protection retirés ; et
- des moyens (20, 22) de commande de la mise en marche/arrêt desdits moyens de dépression,
comprénaur:
- au moins un diaphragme (34) de forme adaptable au contour du membre revêtu du vêtement de protection et agissant par pression sur ledit membre pous assurer une étanchéité de la boîte d'enlèvement par rapport à l'extérieur, ledit diaphragme étant apte à prendre une pluralité de positions ; et
- des moyens pour aspirer les vêtements retirés vers le réceptacle.
caractérisé en ce que le diaphragme peut prendre trois positions :
- une position (F) de fermeture, dans laquelle il enserre le membre revêtu du vêtement de protection afin d'obtenir l'étanchéité entre la boîte d'enlèvement et l'extérieur ;
- une position (O) d'ouverture, dans laquelle il permet le passage du membre vers ou hors de la boîte d'enlèvement ; et
- une position (I) intermédiaire de transition, dans laquelle il permet un passage contrôlé de l'air entre l'extérieur du membre et l'intérieur du vêtement de protection.

2. Dispositif selon la revendication 1, caractérisé en ce que les moyens pour aspirer les vêtements comportent un obturateur (17) amovible situé à la jointure de la chambre de réception et de la canalisation, et assurant dans une première position, une fermeture entre ladite chambre de réception et ladite canalisation et, dans une seconde position, une ouverture entre lesdites chambre et canalisation.

3. Dispositif selon l'une quelconque des revendications 1 à 2 , caractérisé en ce que les moyens pour aspirer comportent en outre un opercule (5), commandé par un moteur, permettant de fermer hermétiquement la boîte d'enlèvement lors de l'aspiration des vêtements vers le réceptacle.

4. Dispositif selon l'une quelconque des revendications 1 à 3, caractérisé en ce que les moyens pour aspirer les vêtements de protection comportent en outre une vanne (13) assurant un passage de l'air entre l'extérieur et la boîte d'enlèvement pour la mise à pression normale dans ladite boîte.

5. Dispositif selon l'une quelconque des revendications 1 à 4, caractérisé en ce que la canalisation comporte, à son embouchure dans le réceptacle, un compteur de vêtements permettant d'envoyer une information pour prévenir lorsque le réceptacle est plein.

## Claims

1. Device for removing a protective garment slipped onto a limb of the body of a user, this device comprising:
- at least one box (4) for removing the said garment, including a receiving chamber (28) into which the limb clad with the garment is introduced, a vacuum chamber (26) and a perforated wall (30) located between the said chambers;
- vacuum means (8) for creating a pressure difference in the receiving chamber with respect to the normal pressure outside the removal box;
- a receptacle (6) connected via a pipe (10) to the receiving chamber, in which receptacle are stored the removed protective garments; and
- means (20, 22) for actuating the turning-on/turning-off of the said vacuum means,
comprising
- at least one diaphragm (34) of shape which can match the contour of the limb clad with the protective garment and acting by pressing on the said limb in order to seal the removal box with respect to the outside, the said diaphragm being capable of adopting a plurality of positions; and
- means for sucking the removed garments away to the receptacle,
characterized in that the diaphragm can adopt three positions:
- a closed position (F), in which the diaphragm grips the limb clad with the protective garment so as to obtain the seal between the removal box and the outside;
- an open position (O), in which the diaphragm allows the limb to pass towards or out of the removal box; and
- an intermediate transition position (I), in which the diaphragm allows controlled passage of the air between the outside of the limb and the inside of the protective garment.

2. Device according to Claim 1, characterized in that the means for sucking away the garments include a removable shutter (17) located at the junction of the receiving chamber and the pipe and providing, in a first position, a closure between the said receiving chamber and the said pipe and, in a second position, an opening between the said chamber and the said pipe.

3. Device according to either of Claims 1 and 2, characterized in that the sucking means furthermore include a cover (5), driven by a motor, making it possible to seal hermetically the removal box as the garments are being sucked away to the receptacle.

4. Device according to any one of Claims 1 to 3, characterized in that the means for sucking away the protective garments furthermore include a valve (13) providing passage of the air between the outside and the removal box for normal pressurization in the said box.

5. Device according to any one of Claims 1 to 4, characterized in that the pipe includes, at its outlet in the receptacle, a garment counter making it possible to send information in order to give a warning when the receptacle is full.

## Patentansprüche

1. Vorrichtung zum Ausziehen eines über ein Körperglied eines Benutzers gestreiften Schutzkleidungstücks, wobei diese Vorrichtung umfaßt:
- wenigstens einen Kasten (4) zum Ausziehen dieses Kleidungsstücks, der eine Aufnahmekammer (28) enthält, in die das mit dem Kleidungsstück versehene Körperglied eingeführt wird, sowie eine Unterdruckkammer (26) und eine perforierte Wand (30), die sich zwischen besagten Kammern befindet;
- Unterdruckeinrichtungen (8), um einen Druckunterschied in der Aufnahmekammer zu erzeugen, bezogen auf den Normaldruck außerhalb der Aufnahmekammer;
- einen Behälter (6), über einen Kanal (10) mit der Aufnahmekammer verbunden, in dem die ausgezogenen Schutzkleidungsstücke gelagert werden; und
- Einrichtungen (20, 22) zur Betätigung des Einschaltens/Ausschaltens der genannten Unterdruckeinrichtungen,
- wenigstens eine Membran (34), die sich der Form des mit dem Schutzkleidungsstück versehenen Körperglieds anpaßt und mittels Druck auf besagtes Körperglied einwirkt, um eine Dichtheit des Ausziehkastens bezüglich der Außenseite zu gewährleisten, wobei besagte Membran eine Vielzahl von Stellungen einnehmen kann; und
- Einrichtungen zum Absaugen der ausgezogenen Kleidungsstücke in Richtung Behälter,
**dadurch gekennzeichnet,**
daß die Membran drei Stellungen einnehmen kann:
- eine Verschlußstellung (F), in der sie das mit dem Schutzbekleidungsstück versehene Körperglied umschließt, um Dichtheit herzustellen zwischen dem Ausziehkasten und der Außenseite;
- eine öffnungsstellung (O), in der sie den Durchgang des Körperglieds in den Ausziehkasten hinein oder aus ihm heraus ermöglicht; und
- eine Zwischenstellung (I) des Übergangs, in der sie einen kontrollierten Luftdurchlaß zwischen der Außenseite des Körperglieds und der Innenseite des Schutzbekleidungsstücks ermöglicht.

2. Vorrichtung nach Anspruch 1, dadurch gekennzeichnet, daß die Einrichtungen zum Absaugen des Kleidungsstücks einen wegklappbaren Verschluß (17) umfassen, der sich an der Verbindungsstelle der Aufnahmekammer und des genannten Kanals befindet und der in einer ersten Stellung einen Verschluß bildet zwischen Aufnahmekammer und Kanal und in einer zweiten Stellung eine Öffnung zwischen besagter Kammer und dem Kanal.

3. Vorrichtung nach einem der Ansprüche 1 und 2, dadurch gekennzeichnet, daß die Einrichtungen zum Absaugen außerdem einen Deckel (5) umfaßt, betätigt durch einen Motor, um beim Absaugen der Kleidungsstücke in Richtung Behälter den Ausziehkasten hermetisch verschließen zu können.

4. Vorrichtung nach einem der Ansprüche 1 bis 3, dadurch gekennzeichnet, daß die Einrichtungen zum Absaugen der Schutzkleidungsstücke außerdem ein Ventil (13) umfassen, das zur Herstellung des Normaldrucks in dem Ausziehkasten einen Luftdurchlaß zwischen der Außenseite und dem Ausziehkasten ermöglicht.

5. Vorrichtung nach einem der Ansprüche 1 bis 4, dadurch gekennzeichnet, daß der Kanal an seiner Mündung in den Behälter einen Kleidungsstückezähler umfaßt, der eine Information senden kann, um zu melden, wenn der Behälter voll ist.
